# EUROPEAN PATENT SPECIFICATION

(11) **EP 1 663 496 B1**
(45) Date of publication and mention of the grant of the patent: **28.09.2011**
(21) Application number: 04713971.2
(22) Date of filing: 24.02.2004
(51) Int. Cl.: B01L 3/14, A61B 5/15

(54) **HEAT-SEALED CAP FOR RESEALING TEST TUBES IN CLINICAL ANALYSES**
HEISSGESIEGELTE VERSCHLUSSKAPPE ZUM WIEDERVERSCHLIESSEN VON IN DER KLINISCHEN ANALYSE VERWENDETEN TESTRÖHRCHEN
BOUCHON THERMOSCELLE POUR REFERMETURE HERMETIQUE DE TUBES DE TEST POUR ANALYSES CLINIQUES

(30) Priority: 18.09.2003 IT BO20030542
(43) Date of publication of application: 07.06.2006
(73) Proprietor: ECOCAP'S S.r.l., 40033 Casalecchio di Reno(Bologna) (IT)
(72) Inventor: INNOCENTI, Carlo, I-40128 Bologna (IT)
(74) Representative: Trentini, Ermanno
(86) International application number: PCT/IT2004/000082
(87) International publication number: WO 2005/025750

(56) References cited:
- EP-A- 0 059 297
- EP-A- 0 352 322
- EP-A- 0 353 710
- EP-A- 0 373 667
- GB-A- 1 134 433
- GB-A- 1 388 494
- US-A- 5 370 253
- US-B1- 6 382 441

## Description

### TECHNICAL FIELD

The present invention pertains to an aluminium cap for resealing test tubes for chemical analyses after they have undergone processing in automatic or manual clinical chemical analysers.

### BACKGROUND ART

It is known that the analysis of bodily fluids usually takes place in a general clinical laboratory; the sample, for example blood taken from a vein, is collected in a test tube which is capped and labelled and then sent to a laboratory, where the contents are analysed using suitable apparatuses, with or without the addition of reagents.

At this point the analysis could be considered terminated if challenges to or doubts regarding the results of the analyses did not arise in a significant percentage of cases.

In fact, it may occur that analyses have to be repeated, which means that the sample must be preserved for a given number of days prior to its disposal. Samples may also be preserved in the event of contestation by patients or, more generally speaking, for medical-legal reasons.

Test tubes containing already analysed samples, obviously without the original seal, plug or screw cap, previously removed to enable penetration of the sampling needle, are collected in multiple supports which are packed and placed in special cold storage compartments. Therefore, the prevention of fluid spills is left up to the adherence of plastic wrap or precarious coverings wrapped all around the support. The risk of spillage or leaks in the event of an accident is thus very high, as is the risk of personnel being contaminated by sometimes dangerously infected samples.

With reference to background art, there is no clear evidence of caps used for re-sealing of test tube, even if, patents GB-A-1 388 494, EP-A-O 353 710, EP-A-O 059 297, US-B 6 382 441, show claims relevant to caps for sealing with similar but not identical characteristics.

### DISCLOSURE OF INVENTION

To avoid this and other problems, a system has been devised to reseal test tubes of any type and size containing already analysed samples, after the analysis itself and prior to storage, in order that the test tube container can be handled in complete safety. This solution is obviously also useful for the disposal of waste from hospitals or laboratories which, when open test tubes are involved, can create hazards for personnel who manually transfer such products from the laboratory to waste containers. These and other aims are accomplished by the device of the present invention, basically consisting in an aluminium cap to be applied on the mouth of the test tube;the cap is composed of a square piece of aluminium foil, such as to adhere, following a heat sealing process, to the edge or side or both test tube surfaces, thereby sealing it perfectly. The adhesion of the aluminium cap to the plastic of the test tube is achieved thanks to special head-sealed lacquer present on the contact surface of the pre-treated aluminium foil. A similar lacquer is also applied on the top surface of the cap so that in the unlikely, but not impossible event of the sample undergoing further analyses, the same test tube can again be perforated using the sampling needle and later resealed.

The invention is correctly defined in the claim.

### BRIEF DESCRIPTION OF DRAWINGS

These and other characteristics will now be further clarified in relation to a simple design configuration of the invention. The description herein serves purely illustrative purposes and does not restrict the scope of this patent.

Reference is made to the enclosed drawings in which:
Fig. 1 Approximately illustrates the sequence of operations.

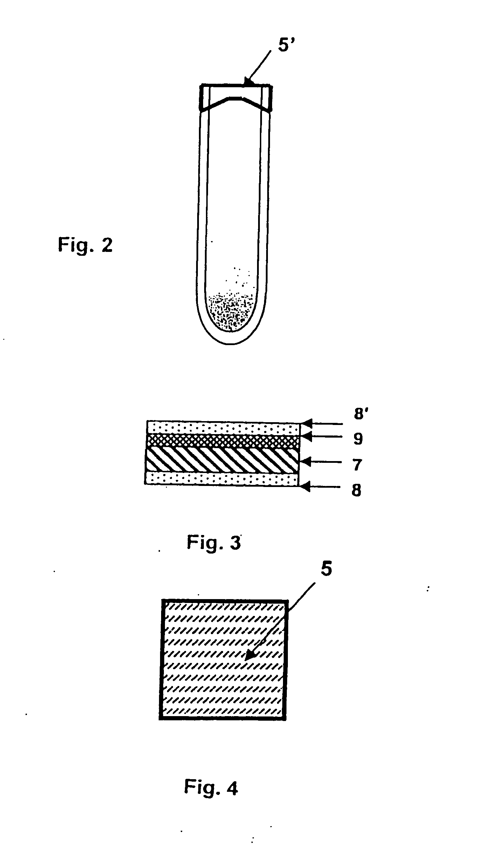

Figs. 2-3-4 Show the aluminium foil applied on a test tube, viewed in cross section and from above.

### BEST MODE FOR CARRYING OUT THE INVENTION

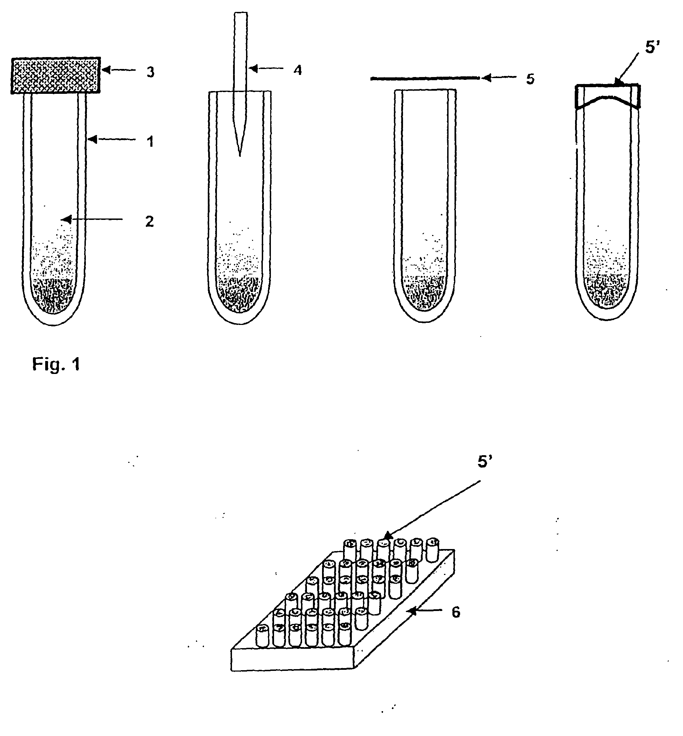

1 indicates the test tube containing the sample 2, 3 the original cap, still Fig.1 shows the test tube with its original cap removed so that the sampling needle 4 can penetrate it; again Fig.1 shows the analysed test tube surmounted by cap 5; finally Fig.1 shows the test tube resealed by cap 5', sealed and possibly moulded over the mouth. 6 indicates the container of resealed test tubes ready for refrigeration, 7 the layer of aluminium forming the body of the cap, 8 the bottom layer of lacquer, 9 the ink layer of the stamp, 8' the top layer of lacquer.

## Claims

1. Heat-sealed aluminium cap for resealing test tubes made of plastic with a rounded-edge or threaded mouth for clinical analyses, said cap comprising:
a cap body in symmetrical shape such as to cover the open mouth of the test tube and wrap over part of the lateral surface thereof, said cap body consisting of a thin deformable foil (7) to be placed and sealed on the uncapped mouth of the test tube (1), the cap further comprising a first layer of heat-sealing adhesive lacquer (8) on the surface of the cap in contact with the mouth of the test tube, said lacquer being compatible with the material of which the test tube itself is made,
**characterized in that** the cap body consists of square piece of aluminium foil (7), wherein a second layer of lacquer (8') is applied on the top surface of the cap in direct contact with the aluminum foil (7) or above a stamp (9) when present on the aluminium foil (7).

## Patentansprüche

1. Heißversiegelte Aluminiumkapsel zur Wiederversiegelung von Kunststoff-Reagenzgläsern für klinische Analysen mit abgerundetem oder Gewinderand.
Diese Kapsel umfasst einen Kapselkörper mit einer solchen symmetrischen Form, dass sie die offene Reagenzglasmündung bedeckt und einen Teil der Seitenfläche umfasst, wobei der Kapselkörper eine dünne und verformbare Folie (7) umfasst, die auf die offene Reagenzglasmündung (1) gelegt und angeschweißt wird. Die Kapsel umfasst zudem auf der Kapseloberfläche, in Kontakt mit der Reagenzglasmündung, eine erste Schicht Siegellack (8) aus einem mit dem Reagenzglasmaterial kompatiblen Material, **gekennzeichnet dadurch, dass** der Kapselkörper eine dünne, quadratische Aluminiumfolie (7) umfasst, wo eine zweite Lackschicht (8') auf der Kapseloberseite in direktem Kontakt mit der Aluminiumfolie (7) oder oberhalb der Stempelung (9), falls auf der Aluminiumfolie (7) eine Stempelung vorhanden ist, angebracht wird.

## Revendications

1. Capsule en aluminium thermo-soudée pour le re-scellage d'éprouvettes réalisées en matériau plastique à bord rond ou fileté pour analyses cliniques.
Cette capsule comprend un corps capsule de forme symétrique permettant de recouvrir l'ouverture de l'éprouvette et d'envelopper partiellement la superficie latérale, le corps capsule comprend une feuille fine et déformable (7) à placer et sceller sur l'ouverture débouchée de l'éprouvette (1). La capsule comprend également une couche de laque adhésive thermoscellante (8) située sur la surface de la capsule au contact de l'ouverture de l'éprouvette, compatible avec le matériau dont l'éprouvette est constituée, **caractérisée en ce que** le corps capsule comprend une fine feuille d'aluminium de forme carrée (7), sur laquelle est appliquée une deuxième couche de laque (8') sur la surface supérieure de la capsule en contact direct avec la feuille d'aluminium (7) ou sur le poinçonnage (9) dans le cas où le poinçonnage serait présent sur la feuille d'aluminium (7).
